Europäisches Patentamt

European Patent Office

Office européen des brevets

(19)

(11) EP 0 717 753 B1

(12) EUROPEAN PATENT SPECIFICATION

(45) Date of publication and mention
of the grant of the patent:
**02.12.1998 Bulletin 1998/49**

(21) Application number: **94927569.7**

(22) Date of filing: **06.09.1994**

(51) Int Cl.6: **C07K 14/415**, A61K 47/48,
C12P 21/02

(86) International application number:
**PCT/EP94/02969**

(87) International publication number:
**WO 95/07297 (16.03.1995 Gazette 1995/12)**

(54) **RIBOSOME INACTIVATING PROTEINS EXTRACTED FROM SEEDS OF SAPONARIA OCYMOIDES AND VACCARIA PYRAMIDATA, THEIR PREPARATION AND IMMUNOTOXINS CONTAINING THEM**

RIBOSOM-INAKTIEVIRENDE PROTEINE, DIE AUS SAPONARIA OCYMOIDES UND VACCARIA PYRAMIDATA SAMEN EXTRAHIERT SIND SOWIE DEREN HERSTELLUNG UND IMMUNOTOXINE, DIE SIE ENTHALTEN

PROTEINES D'INACTIVATION DE RIBOSOMES EXTRAITES DE GRAINES DE SAPONARIA OCYMOIDES ET DE VACCARIA PYRAMIDATA, LEUR PREPARATION ET IMMUNOTOXINES LES CONTENANT

(84) Designated Contracting States:
**DE ES FR GB IT**

(30) Priority: **06.09.1993 IT FI930171**
**06.09.1993 IT FI930172**

(43) Date of publication of application:
**26.06.1996 Bulletin 1996/26**

(73) Proprietor: **Menarini Ricerche S.p.A.**
**00040 Pomezia (IT)**

(72) Inventors:
* **STIRPE, Fiorenzo**
**I-40125 Bologna (IT)**
* **BOLOGNESI, Andrea**
**I-40033 Casalecchio Di Reno (IT)**
* **MELE, Antonio**
**I-51016 Montecatini Terme (IT)**
* **DE SANTIS, Rita**
**I-00040 Pomezia (IT)**

(74) Representative: **Gervasi, Gemma, Dr. et al**
**NOTARBARTOLO & GERVASI Srl,**
**Corso di Porta Vittoria, 9**
**20122 Milano (IT)**

(56) References cited:
**WO-A-90/12034** **WO-A-90/12597**

* **THE EUROPEAN JOURNAL OF CANCER , vol. 27, suppl. 3, 1991, page S57, E. TOSI et al.**
* **THE LANCET, vol. 339, 16 May 1992, LONDON, GB, pages 1195-1196, B. FALINI et al.**
* **INTERNATIONAL JOURNAL OF CANCER, vol. 55, no.1, 19 August 1993, pages 122-127, R. TECCE et al.**
* **THE BIOCHEMICAL JOURNAL, vol. 216, no. 3, 15 December 1983, pages 617-625, F. STIRPE et al.**
* **BIOCHIMICA ET BIOPHYSICA ACTA, vol. 1154, no. 3/4, 1993, pages 237-282, L. BARBIERI et al.**

**Description**

Field of the invention

The present invention refers to new proteins extracted from the seeds of Saponaria ocymoides and Vaccaria pyramidata respectively, which are able to inactivate ribosome proteins, their preparation and immunotoxins (immunoconjugates) containing such proteins and an antibody, or other suitable carriers, useful in the treatment of tumors.

State of the art

It is known that many plant tissues contain proteins which are able to inhibit the protein synthesis by inactivating the eukaryotic ribosomes. These proteins, usually named RIP (Ribosome Inactivating Proteins), are normally present as monomers having a molecular weight of about 30 kDa (RIP type-1) and have an isoelectric point which is often strongly basic.

Considering the fact that all the toxins used up to now induce an immunological response in the patients, the identification and purification of new RIP proteins is extremely important for therapeutical application.

Moreover it is known that, with the aim of obtaining molecules which are selectively cytotoxic, many of the known RIP were linked to molecular vectors able of carrying them on specific cell populations constituing the wanted target. Normally to prepare compounds having a specific cytotoxic action monoclonal antibodies are used as protein vectors (immunotoxins), anyhow also hormones, Growth Factors and lectins were used as vectors in the therapeutical treatment of tumors.

For the therapeutical application the link between antibody and toxin should be strong enough to remain stable during the passage of the immunotoxin in the tissues up to the target cell and should be broken once inside the cell in order to develop its inactivating action.

Monoclonal antibodies specific for EGF-r (Epidermal Growth Factor Receptor) were used in the art to construct immunoconjugates capable of reducing the metastatic potential of tumoral cells transplanted in athymic mice.

Since EGF-R is expressed also by healthy cells it can not be considered qualitatively tumor-specific but an abnormal quantity can be exploited for the wanted purposes. Therefore in order to take advantage of the quantitative difference of expression of EGF-R between healthy and tumoral cells it is necessary to provide reagents capable of reacting selectively only with cells showing an high expression of the factor.

Description of the figures

Figure 1 shows the elution profile of the protein obtained from Saponaria ocymoides by ion exchange chromatography on CM-Sepharose column (81.0 cm x 20 cm), prepared with sodium-phosphate buffer 5 mM (pH 7) eluted with a linear gradient 0 - 0.3 M NaCl in the same buffer.

In the abscissa the elution volumes are reported while in ordinate the inhibition of the protein synthesis (%) is shown. The line — is the absorbance, the line —□— shows the protein inhibition (%) in respect of control.

Figure 2 shows the elution profile of the protein obtained from Vaccaria pyramidata by ion exchange chromatography on CM-Sepharose column (1.6 cm x 20 cm), prepared with sodium-phosphate buffer 5 mM (pH 7) eluted with a linear gradient 0,05 - 0.3 M NaCl in the same buffer.

In the abscissa the elution volumes are reported while in ordinate the inhibition of the protein synthesis (%) is shown. The line — is the absorbance, the line —□— shows the protein inhibition (%) in respect of control.

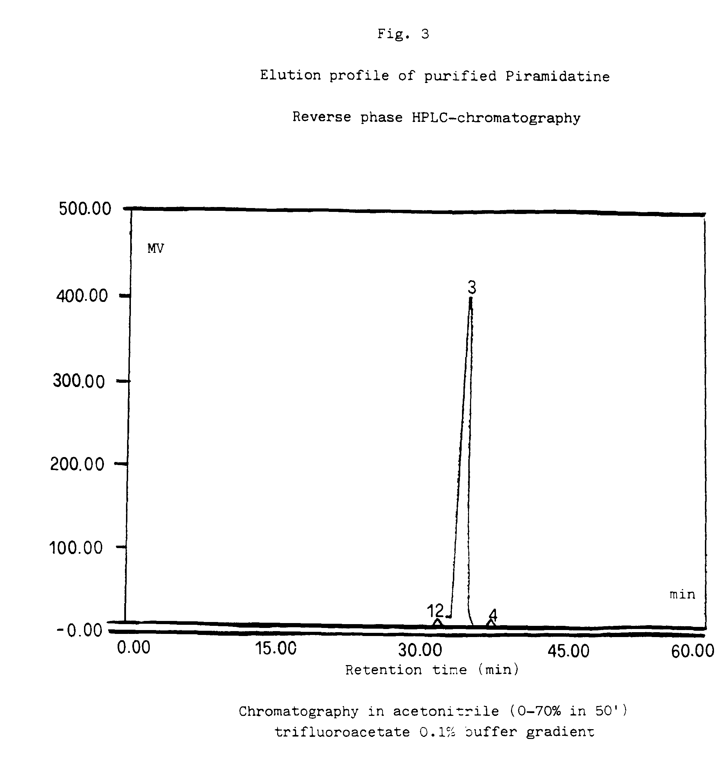

Figure 3 shows the elution profile of Piramidatine purified by reverse phase HPLC.

Figure 4 shows the elution profile of Ocymoidine purified by reverse phase HPLC. Figure 5 shows the electrophoresis SDS-PAGE of the imunoconjugates in non-reducing environment (Phast System, Pharmacia; Phastgel Gradient 4 - 15%)

Figure 6 shows the inhibition of the protein synthesis on a lysate of rabbit reticulocytes by immunoconjugates MGR-6-Piramidatine (A) and MGR-6-Ocymoidine (B).

On the ordinate the inhibition in % is reported and on the abscissa the toxin concentration.

Figure 7 shows the inhibition of the protein synthesis on a lysate of rabbit reticulocytes by immunoconjugates Mint5-Piramidatine (A) and Mint5 - Ocymoidine (B).

On the ordinate the inhibition in % is reported and on the abscissa the toxin concentration.

- SEQ ID NO:1 shows the nucleotide and amino acid sequence of the variable region of the heavy chain of antibody Mint5. The nucleotide sequences of eight codons at the end 5' and 11 codons at the end 3' are imposed by the primers used in the amplification of the cDNA by PCR. The primers were chosen according to the work of Orlandi et al., PNAS, 86:3833-3837 (1989). The sequences reported as: CDR1, CDR2 and CDR3 (CDR = complementary determining region) refer to the iper-variable regions.

- SEQ ID NO:2 shows the amino acid sequence (deduced from the nucleotide sequence of SEQ ID NO:1) of the variable region of the heavy chain of antibody Mint5
- SEQ ID NO:3 shows the N-terminal fragment of the amino acid sequence of the variable region of the heavy chain. Such sequence was determined by Edman's degradation. Only the amino acids 1, 3, 5 and 6 are different in respect of the amino acid chain deduced and reported under SEQ ID NO:2. The amino acids reported as Xaa were not identified correctly.
- SEQ ID NO: 4 shows the nucleotide and amino acid sequence of the variable region of the light chain of antibody Mint5. The nucleotide sequence of eight codons (24 nucleotide) at the 5' and 3' ends were imposed by the primers used in the amplification of cDNA by PCR. The sequences indicated with: CDR1, CDR2 and CDR3 refer to the ipervariable regions.
- SEQ ID NO:5 shows the amino acid sequence (deduced from the nucleotide sequence of SEQ ID NO:4) of the varable region of the light chain of the antibody Mint5.
- SEQ ID NO:6 shows the N-terminal fragment of the amino acid sequence of the variable region of the light chain of antibody Mint5. Such sequence was determined by Edman's degradation. Only the amino acids 3 and 8 are different in respect of the amino acid chain deduced and reported under SEQ ID NO:5.
- SEQ ID NO:7 shows the amino acid sequence of the RIP obtained from Saponaria ocymoides.
- SEQ ID NO:8 shows the sequence from amino acid 38 to 73 of the RIP obtained from Vaccaria pyramidata.
- SEQ ID NO:9 shows the sequence from amino acid 112 to 123 of the RIP obtained from Vaccaria pyramidata.
- SEQ ID NO:10 shows the sequence from amino acid 235 to 258 of the RIP obtained from Vaccaria pyramidata.
- SEQ ID NO:11 shows the sequence from 265 to 277 of the RIP obtained from Vaccaria pyramidata.

<u>Detailed description of the invention</u>

It is an object of the present invention new RIP proteins extracted from Saponaria ocymoides and Vaccaria pyramidata.

The invention refers also to immunoconjugates formed by the above said proteins and monoclonal antibodies, more particularly the antibodies are antibodies specific against EGF-R (Mint5) and p185 HER2/neu (MGR-6). The present invention refers also to the use of the immunoconjugates for the preparation of pharmaceutical compositions useful in the therapy of tumors, autoimmunity, infections, antiviral therapy and transplant rejection.

The present invention refers also to a method for the treatment of tumors wherein a therapeutically active amount of the immunoconjugates according to the invention is administered to the patient.

The RIP according to the invention are hereinafter indicated as Ocymoidine (the one extracted from Saponaria ocymoide) and Piramidatine (the one extracted from Vaccaria pyramidata).

The Rip according to the invention were purified through a method comprising, in sequence: ion exchange chromatography on S-Sepharose and CM-Sepharose followed by hydrophobic interaction chromatograpy on Phenyl-Sepharose (Ocymoidine) and filtration on Amicon PM10 membrane (Piramidatine).

Both proteins have a purity > 98% by reverse phase HPLC.

The proteins have a molecular weight (in SDS-PAGE) of about 30,000 Da (Ocymoidine) and about 24,000 Da (Piramidatine) and a strongly basic isoelectric point (about 9.5). Their amino acid composition was determined.

The purified proteins show an inhibition of 50% ($IC_{50}$) of the protein synthesis in an acellular system of rabbit reticulocyte lysate in concentration of 1 -2 ng/ml ($IC_{50} < 10^{-10}$M).

As above said the present invention refers also to the immunoconjugates formed by the above said proteins Ocymoidine and Piramidatine with antibodies.

In particular are described immunoconjugates formed by monoclonal antibodies and more in particular antibodies specific agaìnts EGF-R and pl85HER2/neu, such monoclonal antibodies are hereinafter indicated as Mint5 and MGR6 respectively.

Mint5 is a monoclonal antibody specific for EGF-R obtained through immunization of mice with the cell line A431 (Epidermoid carcinoma cell line over-expressing EGF receptors).

Mint5 was deposited with the DSM (Deutches Sammlung von Mikroorganismen un Zellkulturen GmbH) under the number ACC2150.

Mint5 is able of discriminating between cells having normal and abnormally high expression of EGF-R. The identification threshold of Mint5 in immunocytochemistry tests is $5x10^4$ receptor/cell (10 μm diameter). Mint5 can inhibit the linking of EGF-R to the receptor on target cells and conversely its bonding is inhibited by EGF. Therefore Mint5 can recognize in the receptor the linking situ of EGF.

Mint5 inhibits the growth of cells having high levels of EGF-R expression <u>in vivo</u> and <u>in vitro</u> but has no effect on cells expressing normal levels of receptors.

After its binding to A431 cells, Mint5 easily enters the cell and is able to inhibit the growth of tumoral cells in transplanted athymic mice.

In the same way as Mint5, also MGR6 [described by Centis F. et al. Hybridoma 11(3): 267 - 276 (1992)] is able to identify a protein (p185 HER2/neu) expressed at high level on tumoral cells. This protein is the product of gene c-erbB2 expressed also in normal cells and present in chromosoma 17 codifying a protein highly homologous, but different, to EGF-R.

In normal breast cells c-erbBe is present as a single gene pair but is amplified 25 - 30% times in primary breast tumors.

MGR6 is a monoclonal antibody (IgG2/K) obtained by immunization of BALB/c mice with cells of lung carcinoma CALU3 which overexpress p185 HER2/neu.

Therefore MGR6, being able of discriminating between cells having normal and abnormally high values of expression of p185 HER2/neu and being able to enter easily the cell, is a useful vector for toxin in the antitumor therapy. The immunoconjugates according to the invention were prepared according to the method described by Thorpe et al.: "Monoclonal Antibody - Toxin conjugates: Aiming the magic bullet" in Monoclonal Antibodies in Clinical Medicine, Academic Press, pp.168 - 190 (1982).

This method is based on the use of hetero-bifunctional reagents as N-succinimidyl-3-(2-pyridylthio)-propionate (SPDP), 2-iminothiolane (Traut reagent), S-acetyl-mercaptosuccinic anhydride (SAMSA), carbodiimide or glutaraldehyde or the like. These reagents are used according to standard methods, in particular 2-iminothiolane is used to activate the antibody and the toxin which will be thereafter conjugated by a disulfide bridge.

Immunoconjugates prepared with this method were previously used in clinical studies of the treatment of Hodgkin's lymphoma resistant to traditional treatment, with substantial remission [Falini et al. Response of refractory Hodgkin's disease to monoclonal anti-CD30 imunotoxin; Lancet 339: 1195-1196 (1992)].

In order to increase the stability of the compounds according to the invention their preparation by recombination-techniques were investigated.

Many researchers produced fusion protein in a single polypeptide chain wherein the gene codifying the variable regions of the antibody are linked by a linker-sequence of DNA to the codifying gene for the toxin. The advantage of these molecules, a part from their chemical stability, are a consequence of their little dimension which let foresee a good pharmacokinetic and a reduced immunogenicity in respect of chemical immunoconjugates. Moreover small toxins can enter more easily the tissues and therefore be more efficient against solid tumors.

Example

Preparation and purification of Pirimidatine and Ocymoidine

Vaccaria pyrimidata and Saponaria ocymoides seeds are homogenized with an Ultraturrax homogenizer in 8 volumes of cold PBS buffer (pH 7.15) (0.14 M NaCl/5 mM sodium phosphate). The suspension is extracted during 12 hours at 4°C under magnetic stirring, filtered and clarified by centrifugation at 30.000 rpm for 30 minutes at 4°C. The raw extracts are acidified with glacial acetic acid up to pH 4.0 - 4.1 and centrifuged as above said.

The supernatants are applied to a S-Sepharose column (15 x 2 cm) prepared with 20 mM sodium acetate (ph 4.5). The column is washed with one volume of the above said buffer and thereafter with sodium-phospate 5 mM (pH 7.15). Proteins were eluted with NaCl 1 M in the same buffer and dialyzed against sodium-phosphate buffer 5 mM (pH 7.15) (Piramidatine) or against the same buffer containing 50' mM NaCl (Ocymoidine) at 4°C.

After centrifugation as above said, in order to recover the precipitate, the protein solution is applied to a CM-Sepharose FF column (22 x 1.6 cm) prepared with the corresponding dialysis buffer; the column is washed with the same buffer up to an absorbance lower than 0.1.

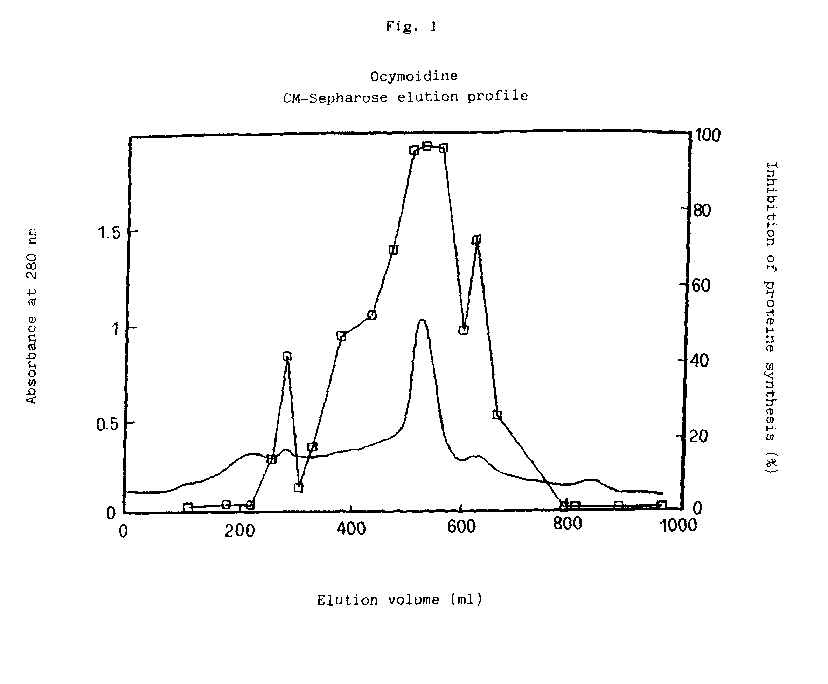

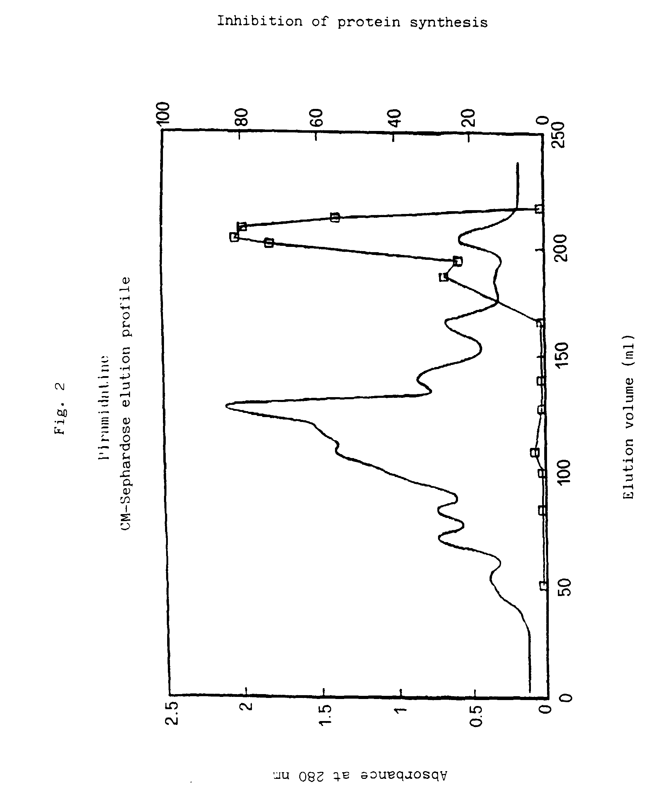

The elution of the adsorbed proteins is performed with 240 ml linear gradient from 0 to 300 mM of NaCl for Pirimidatine (see Fig. 1) and 1 l linear gradient from 50 to 300 mM for Ocymoidine (see Fig. 2).

The fractions inhibiting the protein synthesis in a lysate of rabbit reticulocyte [according to Stirpe et al., 1983. Biochem. J.: 216, 617 - 625; Allen e Scweet, 1962, J. Biol. Chem. 237, 760 - 7] are pooled and dialyzed against water.

To eliminate low molecular weight contaminants the solution of RIP from Vaccaria pyramidata was concentrated and diluted at least 10 times on an Amicon PM 10[R] membrane, and finally concentrated. The solution from Saponaria ocymoides was purified by hydrophobic interaction chromatography.

The RIP solubilised in 30% (w/v) ammonium-sulphate (pH 7.2) was loaded on a Phenyl-Sepharose[R] column (10 x 1.6 cm) prepared with the same buffer. The column was washed up to an absorbance ($A_{280}$) lower than 0.05 and the adsorbed protein was eluted with PBS. The purification scheme is reported in Tables 1 and 2 together with yields and activities.

Physicochemical properties of Pirimidatine and Ocymoidine

Molecular weight was determined by electrophoresis and gel-filtration. The method according to Laemmli for SDS-PAGE was used with the following molecular weight markers: cytochrome c (12,300). myoglobin (17,200), carbonic anhydrase (30,000), ovalbumin (43,000), BSA (66.250) and ovotransferrin (76-78.000).

Gel-filtration was performed by HPLC with a column TSK-gel G3000SW. prepared with buffer (ph 6.7) 0.1 M sodium sulphate, 0.1 M sodium phosphate and calibrated with ribonuclease A (13,700), chymotrypsinogen A (25,000), ovalbumin (43,000) and BSA (67.000). The molecular weight determined by SDS-PAGE analysis is about 30,000 for Ocymoidine and about 24,000 for Pirimidatine.

The two proteins are 99% homogeneous and by gel-filtration show a molecular weight of about 30,000 (Ocymoidine) and about 29,000 (Piramidatine).

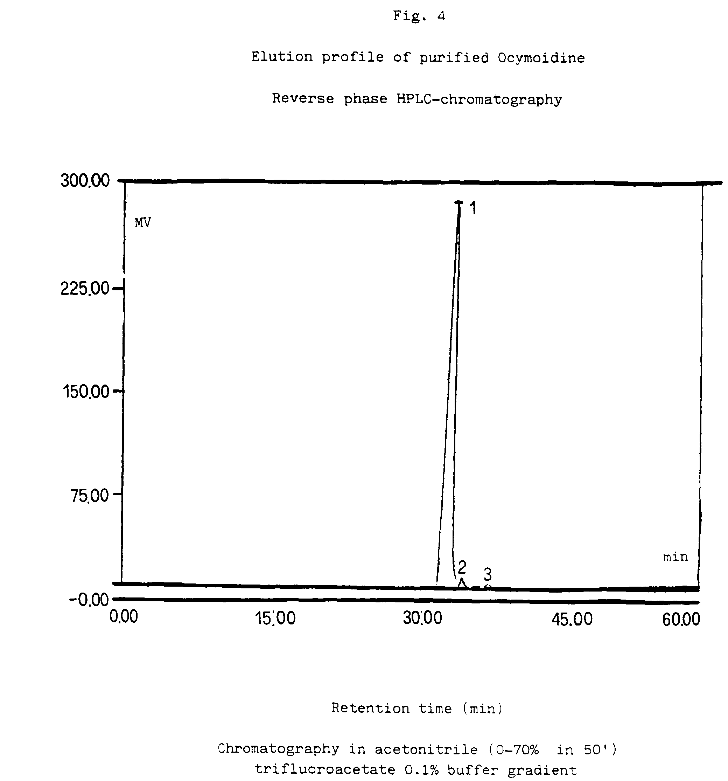

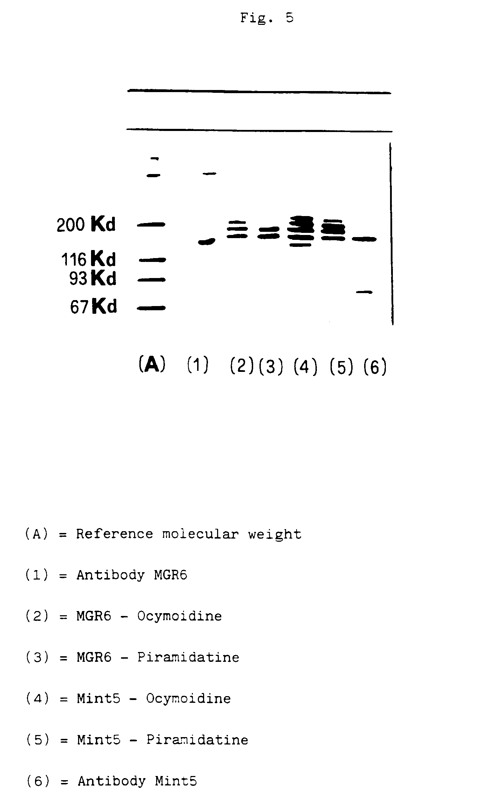

The analysis by reverse phase chromatography on a VYDAC protein C4$^R$ column (25 cm x 4.6 mm) confirmed the high purity of the two proteins so prepared (> 98%) (see Fig. 4 and 5).

The isoelectric point (pI) and the amino acid composition of the proteins were determided as described by Falasca et al. (1982), Biochem. J. 207, 505-509. The isoelectric point is strongly basic (about 9.5).

The physicochemical properties are reported in Tables 3 and 4.

Biological properties of Piramidatine and Ocymoidine

Protein synthesis inhibition

The protein synthesis in a cell-free system was measured as described by Stirpe et al. (see above) with a rabbit reticulocyte lysate according to Allen and Schweet (see above) or by polymerisation of phenylalanine started by Poly (U) with purified ribosomes from rat liver [Montanaro et al.: Biochem. J. 176, 265 - 275 (1978)].

The $IC_{50}$ (concentration giving 50% inhibition) was calculated by linear regression.

In all cases all the $IC_{50}$ were inferior to $10^{-10}$ M (see Tables 1 and 2) and similar to those already described for other RIPs.

Modification of ribosomal RNA

The action of the Rips on rRNA was investigated as described by Endo et al. [J. Biol. Chem.: 262, 5908 - 5912 (1987)]. Samples (0.01 ml) of reticulocytes lysate were incubated in the presence of 1 $\mu$g/ml of RIP for 20 minutes at 37°C. The reaction was stopped by addition of 0.5% SDS. The RNA was extracted with phenol and precipitated with ethanol; in both cases, for the analysis and for the purification of the fragments, the RNA was submitted to electrophoresis on 5% polyacrylammide-gel and the gel was stained with silver as described by Stirpe et al., 1988, Nucleic Acid Res. 16, 1349 - 1357.

Both proteins damaged ribosomal RNA in the same way as ricin and other RIP, as indicated by the fragment which can be obtained after treating with aniline RNA extracted from ribosomes incubated with the RIPs.

The amount of adenine produced by treated ribosomes was determined by reverse phase HPLC on Spherisorb S5 0DS2 (C18)$^R$ column (250 x 4.6 mm) as described by Barbieri et al. 1992, Biochem. J. 286, 1 - 4. The amount of adenine produced was not more than 1 mole for 1 mole of ribosomes (for example 0.82 mol/mol for ribosomes treated with Piramidatine; 0.79 mol/mol for ribosomes treated with Ocymoidine).

Activation of Piramidatine and Ocymoidine

The RIPs were solved up to a concentration of about 3 mg/ml in 50 mM sodium-borate buffer (pH 9). To these solutions traces of the RIPs marked with $^{125}$I (for a total of $10^6$ cpm) were added. After centrifugation to remove corpuscolar material 2-imminothiolane (2-IT) was added up to a final concentration of 1 - 2 mM.

2-IT was solved in 50 mM sodium-borate buffer (pH 9) immediately before use.

After 60' at 28°C solid glycine was added up to a final concentration of 200 mM and after 30' Ellman's reagent, solved in 50 $\mu$l of dimethylformamide immediately before use, was added up to a final concentration of 2.5 mM.

After 30' incubation at 38°C the sample was applied to a Sephadex G25 Coarse$^R$ (25 x 1.6 cm) prepared and eluted with PBS.

The protein peak was collected and the activation ratio was calculated on a little amount of sample diluted 1:5 with PBS after measuring the absorbance at 280 nm and 412 nm and after addition of 1/10 (v/v) of freshly prepared solution of 2-mercaptoethanol 0.22 M.

Activation of antibodies

The monoclonal antibodies Mint5 and MGR-6 in a concentration of 1-6 mg/ml were reacted respectively with 2-IT 0.3 - 0.35 mM, following the above said procedure.

Conjugation

The RIP are concentrated under nitrogen using an Amicon concentrator and reduced adding 1/10 in volume of 2-mercaptoethanol 0.22 M. The reduced toxins were loaded on a Sephadex G25 Coarse$^R$ (25 x 1.6 cm). The protein peak was collected in the concentrator wherein the antibodies were reacting. The reaction mixture was concentrated 4 times under nitrogen, incubated for 20 h at room temperature. The mixture was purified on a Sephacryl S-200 HR$^R$ column (96 x 2.2 cm) prepared and diluted with PBS.

The radioactivity was confronted with the elution profile at 280 nm and the various components purified from the mixture were identified.

The first to elute is the high molecular weight conjugate (HMW), the second the conjugate at low molecular weight (LMW), therafter, in the following order, elute: trimeric RIP (when present), dimeric RIP and non-reacted RIP.

The non-reacted antibodies elute in the last part of the peak LMW having an absorbance of 200 nm. This is shown in the radioactive profile under the UV peak.

The LMW fractions are collected together and the ratio RIP/antibody was calculated for the absorbance at 280 nm and radioactivity of the conjugate LMW and of the non-reacted RIP.

The average conjugation ratio RIP/antibody is compriseed between 0.86 and 1.95.

Following the same procedure the following immunoconjugates were prepared:

MGR-6 - Piramidatine
MGR-6 - Ocymoidine
Mint5 - Piramidatine
Mint5 - Ocymoidine

The conjugation conditions for obtaining the immunotoxins are shown in Table 5.

SDS-PAGE: Control of the antibodies activation

The conjugation degree of Mint5 and MGR-6 with Piramidatine and Ocymoidine was controlled by SDS-PAGE anlysis. As shown in Figure 5 the MGR-6 antibodies (position 1) and Mint5 (position 6) migrate as a single band of about 155 KDa. The corresponding conjugates with piramidatine and ocymoidine (positions 2 - 5) show multiple bands corresponding to the different degrees of conjugation.

Biological properties of the conjugates

Inhibition of protein synthesis in lysate of rabbit reticulocytes

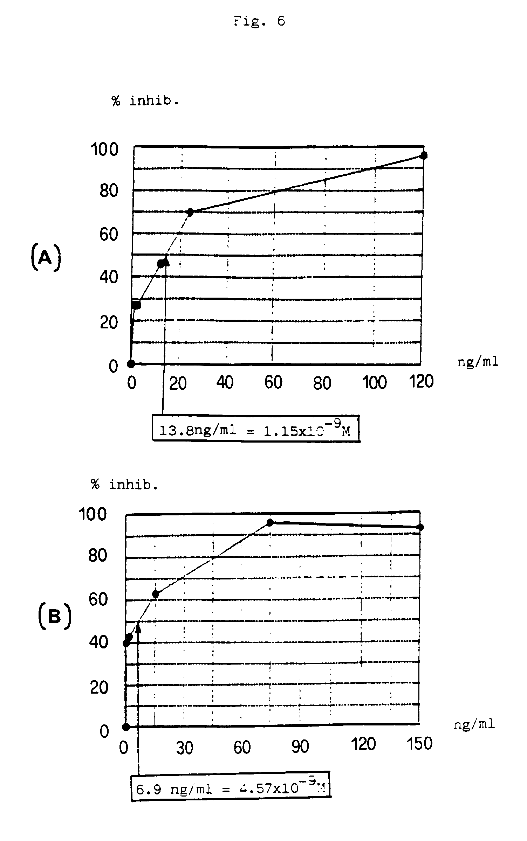

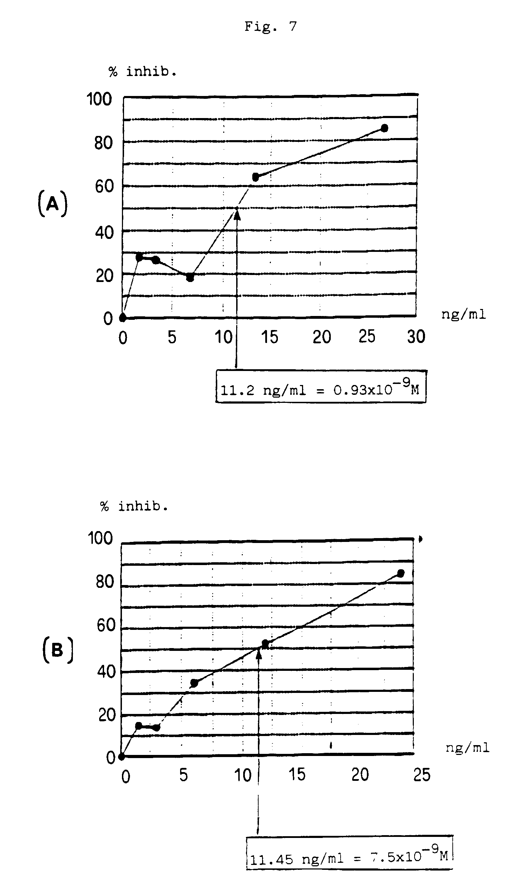

The immunotoxins obtained according to Table 5 were tested for their capacity of inhibiting the protein synthesis in a system of lysate of rabbit reticulocytes. Figures 6 and 7 show that the immunoconjugates MGR-6-Piramiditine, MGR-6-ocymoidine, Mint5-Piramiditine and Mint5-Ocymoidine in a concentration of $10^{-9}$ M (calculated in respect of the toxin content in the immunoconjugate) can inhibit 50% the protein synthesis.

Binding of the immunoconjugates on the target cells

The capacity of the immunoconjugates to bind specifically to the target cells was demonstrated by radioimmunological test. A431 cells (cell line expressing high levels of EGF-R and therefore target for the antibody Mint5) or SKBR3 cells (cell line expressing high levels of p185 HER2/neu and therefore target for the antibody MGR-6) were fixed on a plate with 96 wells and incubated with the antibodies or the immunoconjugates. The binding was demonstrated with a secondary antibody anti-murine immunoglobulins marked with [125]I. The results shown in Table 6 show that the immunoconjugates bind specifically to corresponding cell targets.

Inhibition of cell proliferation and inhibition of protein synthesis on target cells

The inhibition of cell proliferation was performed according to Table 7, following two different procedures reported as protocol A and protocol B, and the results of the toxicity tests are shown in Table 9.

The inhibition of protein synthesis was performed according to Table 8, following two different procedures reported as protocol A and protocol B, and the results of the toxicity tests are shown in Table 10.

Tables 7 and 9 show that when, protocol A is performed, an increase of 100 times of the toxic activity, when the toxin is bound to the antibody, is observed, whilst when protocol B is performed an increase of 1000 times is observed.

Tables 8 and 10 show that when protocol A is performed, an increase of 1000 times of the toxic activity, when the toxin is bound to the antibody, is observed, whilst when protocol B is performed an increase of 10000 times is observed.

## TABLE 1

### ACTIVITY OF RIP FROM SEEDS OF *VACCARIA PYRAMIDATA* (25g)

|  | Total protein mg | $IC_{50}$ ng/ml | Specific activity $U/mg \times 10^3$ | Total activity $U/10^6$ | Activity (yield) % |
|---|---|---|---|---|---|
| Raw material | 939.1 | 148.0 | 6.76 | 6.35 | 100 |
| Acid treatment | 821.5 | 151.0 | 6.62 | 5.44 | 85 |
| S-Seph | 160.5 | 33.1 | 30.21 | 4.85 | 76 |
| CM-Seph | 9.2 | 2.5 | 401.61 | 3.67 | 58 |

## TABLE 2

### ACTIVITY OF RIP FROM SEEDS OF *SAPONARIA OCYMOIDES* (25g)

|  | Total protein mg | $IC_{50}$ ng/ml | Specific activity $U/mg \times 10^3$ | Total activity $U/10^6$ | Activity (yield) % |
|---|---|---|---|---|---|
| Raw material | 698.7 | 24.8 | 40.73 | 28.46 | 100 |
| Acid treatment | 487.2 | 20.9 | 47.87 | 23.32 | 82 |
| S-Seph | 176.8 | 5.0 | 199.60 | 35.28 | 124 |
| CM-Seph | 44.9 | 1.9 | 537.63 | 24.16 | 85 |
| Phenyl-Seph | 31.0 | 1.4 | 740.74 | 22.96 | 81 |

U = Toxin concentration inhibiting (50%) the protein synthesis in 1 ml of the system of rabbit reticulocytes lisate in 5' at 28°C

TABLE 3

| Physicochemical properties of Piramidatine | |
|---|---|
| $M_r$ from gel filtration | 28.000 |
| $M_r$ from SDS-PAGE | 24.500 |
| pI | $\geq 9.5$ |
| $A_{1cm,280}^{1\%}$ | 6.42 |
| Amino acid | Composition (mol/mol) |
| Lysine | 12.1 |
| Histidine | ---- |
| Arginine | 14.8 |
| Aspartic acid/Asparagine | 22.0 |
| Threonine * | 12.0 |
| Serine * | 9.5 |
| Glutamic acid/Glutamine | 19.0 |
| Proline | 6.9 |
| Glycine | 13.9 |
| Alanine | 13.4 |
| Cysteine | ---- |
| Valine | 12.3 |
| Methionine | 2.1 |
| Isoleucine | 11.9 |
| Leucine | 15.1 |
| Tyrosine * | 10.1 |
| Phenylalanine | 8.3 |
| Tryptophan | N.D. |
| Total residues | 183.4 |

N.D.= value not determinable
* The value obtained from hydrolisis at 24, 48 and 72 hours were extrapolated at time zero
$M_r$ = relative molecular weight
A = Absorbance
pI = isoelectric point

TABLE 4

| Physicochemical properties of Ocymoidine | |
|---|---|
| $M_r$ from gel filtration | 30.200 |
| $M_r$ from SDS-PAGE | 30.200 |
| pI | $\geq 9.5$ |
| $A_{1cm,280}^{1\%}$ | 4.80 |
| Amino acid | Composition (mol/mol) |
| Lysine | 22.1 |
| Histidine | 2.7 |
| Arginine | 13.5 |
| Aspartic acid/Asparagine | 33.2 |
| Threonine* | 12.7 |

N.D.= value not determinable
* The value obtained from hydrolisis at 24. 48 and 72 hours were extrapolated at time zero
$M_r$ = relative molecular weight
A = Absorbance
pI = isoelectric point

TABLE 4   (continued)

| Physicochemical properties of Ocymoidine | |
|---|---|
| Amino acid | Composition (mol/mol) |
| Serine * | 13.3 |
| Glutamic acid/Glutamine | 19.0 |
| Proline | 7.8 |
| Glycine | 13.3 |
| Alanine | 20.3 |
| Cysteine | ---- |
| Valine | 14.5 |
| Methionine | 4.8 |
| Isoleucine | 12.4 |
| Leucine | 21.1 |
| Tyrosine * | 9.0 |
| Phenylalanine | 11.9 |
| Tryptophan | N.D. |
| Total residues | 231.6 |

N.D.= value not determinable
* The value obtained from hydrolisis at 24. 48 and 72 hours were extrapolated at time zero
$M_r$ = relative molecular weight
A = Absorbance
pI = isoelectric point

TABLE 5

| Immuno conjugate | Antibody | | RIP | | Immunoconjugate | | | | |
|---|---|---|---|---|---|---|---|---|---|
| | 2IT | conjugated groups | 2IT | conjugated groups | RIP/ Ab | Ab | | RIP | |
| | (mM) | mol/ mol | (mM) | mol/ mol | mol/ mol | µg/ml | Mx10 (-7) | µg/ml | Mx10 (-7) |
| MGR6-Piramidatine | 0.30 | 2.22 | 1.00 | 0.81 | 0.86 | 92.0 | 6.13 | 12.7 | 5.28 |
| MGR6-Ocymoidine | 0.30 | 2.35 | 1.00 | 0.76 | 1.63 | 97.7 | 6.51 | 31.8 | 10.6 |
| Mint5-Piramidatine | 0.35 | 2.40 | 1.00 | 0.80 | 1.95 | 110 | 7.32 | 34.4 | 14.3 |
| Mint5-Ocymoidine | 0.35 | 2.09 | 1.60 | 0.79 | 1.82 | 141 | 9.40 | 51.2 | 17.0 |

TABLE 6

| | % binding | |
|---|---|---|
| | SKBR3 | A431 |
| Mint5 | - | 100 |
| MGR6 | 100 | - |
| Mint5 - Ocymoidine | 9.38 | 78.5 |
| Mint5 - Piramidatine | 18.8 | 110.0 |
| MGR6 - Ocymoidine | 87.5 | 15.3 |
| MGR6 - Piramidatine | 105.2 | 13.0 |

TABLE 7

INHIBITION OF CELL PROLIFERATION: $IC_{50}$

PROTOCOL A:

Cell-seeding on plate
+IT

3H-Thymidine
1mCi/well

48 hs. 37°C          6 hs. 37°C

PROTOCOL B:

Cell-seeding on plate
+IT

Washing

3H-Thymidine
1mCi/well

1 h. 4°C    2 hs. 37°C    48 hs. 37°C    6 hs. 37°C

## TABLE 8

### INHIBITION OF PROTEIN SYNTHESIS
### ON A431 CELLS

PROTOCOL A:

Cell-seeding on plate
+IT

3H-Leucine
1mCi/well

48 hs. 37°C

24 hs. 37°C

PROTOCOL B:

Cell-seeding on plate
+IT

Washing

3H-Leucine
1mCi/well

1 h. 4°C    2 hs. 37°C    48 hs. 37°C    24 hs. 37°C

TABLE 9

| INHIBITION OF CELL PROLIFERATION: IC50 CELL LINE A431 | | | |
|---|---|---|---|
| Protocol | IT | TOX | RATIO |
| A | Mint-Ocym | Ocym | 100 |
|   | $1.9 \times 10^{-12}$ | $2.8 \times 10^{-10}$ | |
|   | Mint-Piram | Piram | 100 |
|   | $1.3 \times 10^{-12}$ | $2.8 \times 10^{-10}$ | |
| B | Mint-Ocym | Ocym | 1000 |
|   | $3.2 \times 10^{-11}$ | $1.5 \times 10^{-8}$ | |
|   | Mint-Piram | Piram | 1000 |
|   | $4.2 \times 10^{-11}$ | $1.5 \times 10^{-8}$ | |
| IT = Immunotoxin TOX = Toxin | | | |

TABLE 10

| INHIBITION OF PROTEIN SYNTHESIS IN CELL SYSTEM: $IC_{50}$ CELL LINE A431 | | | |
|---|---|---|---|
| Protocol | IT | TOX | RATIO |
| A | Mint-Ocym | Ocym | 1000 |
| | $1 \times 10^{-11}$ | $2 \times 10^{-8}$ | |
| | Mint-Piram | Piram | 1000 |
| | $1 \times 10^{-11}$ | $1.5 \times 10^{-8}$ | |
| B | Mint-Ocym | Ocym | 10000 |
| | $1 \times 10^{-11}$ | $2 \times 10^{-7}$ | |
| | Mint-Piram | Piram | 10000 |
| | $1 \times 10^{-11}$ | $2 \times 10^{-7}$ | |
| IT = Immunotoxin TOX = Toxin | | | |

**Claims**

1. Ribosome inactivating protein obtained by extraction from seeds of Vaccaria pyramidata according to the following process:

   a) seeds are homogenized with PBS (pH 7.15, 0.14 M NaCl/5 mM sodium phosphate), centrifuged, acidified with glacial acetic acid up to pH 4.0 - 4.1 and centrifuged;
   b) the supernatant of step (a) is purified with S-Sepharose column prepared with 20 mM sodium acetate (pH 4.5), the column is washed with one volume of the above said buffer and thereafter with 5 mM sodium phosphate buffer (pH 7.15);
   c) the protein is eluted with the same buffer containing also NaCl 1M, dialyzed against 5 mM sodium phosphate buffer (pH 7.15) at 4°C and centrifuged;
   d) the solution from step (c) is loaded on a CM-Sepharose FF column prepared with the dialysation buffer and washed with the same buffer up to an absorbance lower than 0.1 with 240 ml of linear gradient from 0 to 300 mM NaCl;
   e) the fractions presenting inhibiting activity on ribosome are collected and dialyzed against water at 4°C;
   f) the solution is concentrated on Amicon PM 10 membrane (at least 10 times), diluted (at least 10 times) and concentrated;

such protein having an electrophoretic mobility in SDS-PAGE as a double band of about 24.000 Da, molecular weight by gel filtration about 29.000 Da, isoelectric point ≥ 9.5 and the following aminoacid

| composition: | |
|---|---|
| Lysine | 12.1 |
| Histidine | ---- |
| Arginine | 14.8 |
| Aspartic acid/Asparagine | 22.0 |
| Threonine | 12.0 |
| Serine | 9.5 |
| Glutamic acid/Glutamine | 19.0 |
| Proline | 6.9 |
| Glycine | 13.9 |
| Alanine | 13.4 |
| Cysteine | ---- |
| Valine | 12.3 |

(continued)

| composition: | |
|---|---|
| Methionine | 2.1 |
| Isoleucine | 11.9 |
| Leucine | 15.1 |
| Tyrosine | 10.1 |
| Phenylalanine | 8.3 |
| Tryptophan | N.D. |

2. Ribosome inactivating protein obtained by extraction from seeds of Saponaria ocymoides according to the following process:

a) seeds are homogenized with PBS (pH 7.15, 0.14 M NaCl/5 mM sodium phosphate), centrifuged, acidified with glacial acetic acid up to pH 4.0 - 4.1 and centrifuged;

b) the supernatant of step (a) is purified with S-Sepharose column prepared with 20 mM sodium acetate (pH 4.5), the column is washed with one volume of the above said buffer and thereafter with 5 mM sodium phosphate buffer (pH 7.15);

c) the protein is eluted with the same buffer containing also 1 M NaCl, dialyzed against 5 mM sodium phosphate buffer (pH 7.15) at 4°C and centrifuged;

d) the solution from step (c) is loaded on a CM-Sepharose FF column prepared with the dialysation buffer and washed with the same buffer up to an absorbance lower than 0.1 with 1 l of linear gradient from 50 to 300 mM NaCl;

e) the fractions presenting inhibiting activity on ribosome are pooled and dialyzed against water at 4°C

f) purification by hydrophobic interaction chromatography by dissolving the product from step (e) in 30% (p/v) ammonium sulphate, 0.5 M NaCl in 20 mM sodium phosphate (pH 7.15) and treating with a Phenyl-sepharose column prepared with the same buffer, washing up to an absorbance lower than 0.5 and eluting the protein with PBS;

such protein having molecular weight in SDS-PAGE of 30.000 Da, molecular weight by gel filtration 30.000 Da, isoelectric point $\geq$ 9.5 and the following amino acid composition:

| Lysine | 22.1 |
|---|---|
| Histidine | 2.7 |
| Arginine | 13.5 |
| Aspartic acid/Asparagine | 33.2 |
| Threonine | 12.7 |
| Serine | 13.3 |
| Glutamic acid/Glutamine | 19.0 |
| Proline | 7.8 |
| Glycine | 13.3 |
| Alanine | 20.3 |
| Cysteine | ---- |
| Valine | 14.5 |
| Methionine | 4.8 |
| Isoleucine | 12.4 |
| Leucine | 21.1 |
| Tyrosine | 9.0 |
| Phenylalanine | 11.9 |
| Tryptophan | N.D. |

3. Process for the purification of the protein according to claim 1.

4. Process for the purification of the protein according to claim 2.

5. Conjugates of the proteins according to claim 1 and 2 with hormones. lyposomes, monoclonal antibodies, Growth Factors, lectins, cytokines, transferrin and peptides which are fragments of such proteins.

6. Conjugate according to claim 5 wherein the antibody is the monoclonal antibody Mint5. specific for EGF-R.

7. Conjugate according to claim 5 wherein the antibody is the monoclonal antibody MGR6, specific for HER2/neu.

8. Conjugates according to claim 6 and 7 obtained by chemical conjugation or by recombination of the genes of the variable region of the antibody and those codifying for the toxin.

9. Pharmaceutical compositions containing as active principle the proteins according to claims 1 and 2 in combination with pharmaceutically acceptable carriers.

10. Pharmaceutical compositions according to claim 9 for use in anti-viral therapy.

11. Use of the conjugates according to claims 5 - 8 for the preparation of pharmaceutical compositions useful in the therapy of tumors, auto-immunity, infections, antiviral therapy and transplant rejection.

**Patentansprüche**

1. Ribosom-inaktivierendes Protein, erhalten durch Extraktion aus Vaccaria pyramidata-Samen nach dem folgenden Verfahren:

a) die Samen werden mit PBS (pH 7,15, 0,14 M NaCl/5 mM Natriumphosphat) homogenisiert, zentrifugiert, mit Eisessig bis auf pH 4,0 bis 4,1 angesäuert und zentrifugiert;
b) die in der Stufe (a) erhaltene überstehende Flüssigkeit wird mit einer mit 20 mM Natriumacetat (pH 4,5) vorbehandelten S-Sepharose-Kolonne gereinigt, die Kolonne wird mit 1 Volumenteil des obengenannten Puffers und danach mit 5 mM Natriumphosphat-Puffer (pH 7,15) gewaschen;
c) das Protein wird mit dem gleichen Puffer, der auch 1 M NaCl enthält, eluiert, gegen einen 5 mM Natriumphosphat-Puffer (pH 7,15) bei 4°C dialysiert und zentrifugiert;
d) die in der Stufe (c) erhaltene Lösung wird auf eine mit dem Dialyse-Puffer vorbehandelte CM-Sepharose FF-Kolonne aufgegeben und mit dem gleichen Puffer bis zu einer Extinktion von weniger als 0,1 mit 240 ml eines linearen Gradienten von 0 bis 300 mM NaCl gewaschen;
e) die Fraktionen, die eine Ribosom-Inhibierungs-Aktivität aufweisen, werden gesammelt und gegen Wasser bei 4°C dialysiert; und
f) die Lösung wird auf einer Amicon PM 10-Membran eingeengt (mindestens 10-fach), verdünnt (mindestens 10-fach) und eingeengt;

wobei das Protein aufweist eine elektrophoretische Mobilität bei der SDS-PAGE in Form einer Doppel bande von etwa 24 000 Da, ein durch Gelfiltration bestimmtes Molekulargewicht von etwa 29 000 Da, einen isoelektrischen Punkt von > 9,5 und die nachfolgend angegebene Aminosäure-Zusammensetzung:

| Lysin | 12,1 |
|---|---|
| Histidin | -- |
| Arginin | 14,8 |
| Asparaginsäure/Asparagin | 22,0 |
| Threonin | 12,0 |
| Serin | 9,5 |
| Glutaminsäure/Glutamin | 19,0 |
| Prolin | 6,9 |
| Glycin | 13,9 |
| Alanin | 13,4 |
| Cystein | -- |
| Valin | 12,3 |
| Methionin | 2,1 |

(fortgesetzt)

| | |
|---|---|
| Isoleucin | 11,9 |
| Leucin | 15,1 |
| Tyrosin | 10,1 |
| Phenylalanin | 8,3 |
| Tryptophan | n.d. (nicht bestimmt) |

**2.** Ribosom-inaktivierendes Protein, erhalten durch Extraktion aus Saponaria ocymoides-Samen nach dem folgenden Verfahren:

a) die Samen werden mit PBS (pH 7,15, 0,14 M NaCl/5 mM Natriumphosphat) homogenisiert, zentrifugiert, mit Eisessig bis auf pH 4,0 bis 4,1 angesäuert und zentrifugiert;

b) die in der Stufe (a) erhaltene überstehende Flüssigkeit wird mit einer S-Sepharose-Kolonne, die mit 20 mM Natriumacetat (pH 4,5) vorbehandelt worden ist, gereinigt, die Kolonne wird mit 1 Volumenteil des obengenannten Puffers und danach mit 5 mM Natriumphosphat-Puffer (pH 7,15) gewaschen;

c) das Protein wird mit dem gleichen Puffer, der auch 1M NaCl enthält, eluiert, gegen einen 5 mM Natriumphosphat-Puffer (pH 7,15) bei 4°C dialysiert und zentrifugiert;

d) die in der Stufe (c) erhaltene Lösung wird auf eine CM-Sepharose FF-Kolonne, die mit dem Dialyse-Puffer vorbehandelt worden ist, aufgegeben und mit dem gleichen Puffer bis zu einer Extinktion von weniger als 0,1 mit 1 l eines linearen Gradienten von 50 bis 300 mM NaCl gewaschen;

e) die Fraktionen, die eine Ribosom-Inhibierungs-Aktivität aufweisen, werden gesammelt und gegen Wasser bei 4°C dialysiert; und

f) das Produkt der Stufe (e) wird gereinigt durch hydrophobe Wechselwirkungs-Chromatographie durch Auflösen des in der Stufe (e) erhaltenen Produkts in 30 Gew.Nol.-% Ammoniumsulfat, 0,5 M NaCl in 20 mM Natriumphosphat (pH 7,15) und Behandeln mit einer Phenylsepharose-Kolonne, die mit dem gleichen Puffer vorbehandelt worden ist, Waschen bis zu einer Extinktion von weniger als 0,5 und Eluieren des Proteins mit PBS;

wobei das Protein aufweist ein Molekulargewicht bei der SDS-PAGE von 30 000 Da, ein durch Gelfiltration bestimmtes Molekulargewicht von 30 000 Da, einen isoelektrischen Punkt von $\geq$ 9,5 und die folgende Aminosäure-Zusammensetzung:

| | |
|---|---|
| Lysin | 22,1 |
| Histidin | 2,7 |
| Arginin | 13,5 |
| Asparaginsäure/Asparagin | 33,2 |
| Threonin | 12,7 |
| Serin | 13,3 |
| Glutaminsäure/Glutamin | 19,0 |
| Prolin | 7,8 |
| Glycin | 13,3 |
| Alanin | 20,3 |
| Cystein | -- |
| Valin | 14,5 |
| Methionin | 4,8 |
| Isoleucin | 12,4 |
| Leucin | 21,1 |
| Tyrosin | 9,0 |
| Phenylalanin | 11,9 |
| Tryptophan | n.d. (nicht bestimmt). |

**3.** Verfahren zur Reinigung des Proteins nach Anspruch 1.

**4.** Verfahren zur Reinigung des Proteins nach Anspruch 2.

**5.** Konjugate der Proteine nach den Ansprüchen 1 und 2 mit Hormonen, Liposomen, monoklonalen Antikörpern, Wachstumsfaktoren, Lectinen, Cytokinen, Transferrin und Peptiden, die Fragmente solcher Proteine sind.

**6.** Konjugat nach Anspruch 5, worin der Antikörper der monoklonale Antikörper Mint5 ist, der für EGF-R spezifisch ist.

**7.** Konjugat nach Anspruch 5, worin der Antikörper der monoklonale Antikörper MGR6 ist, der für HER2/neu spezifisch ist.

**8.** Konjugate nach den Ansprüchen 6 und 7, die durch chemische Konjugation oder durch Rekombination der Gene der variablen Region des Antikörpers und derjenigen, die für das Toxin codieren, erhalten wurden.

**9.** Pharmazeutische Zusammensetzungen, die als aktives Prinzip (Wirkstoff) die Proteine nach den Ansprüchen 1 und 2 in Kombination mit pharmazeutisch akzeptablen Trägern enthalten.

**10.** Pharmazeutische Zusammensetzungen nach Anspruch 9 für die Verwendung bei der Antiviren-Therapie.

**11.** Verwendung der Konjugate nach den Ansprüchen 5 bis 8 zur Herstellung von pharmazeutischen Zusammensetzungen, die verwendbar sind bei der Therapie von Tumoren, Autoimmun-Erkrankungen, Infektionen, bei der Antiviren-Therapie und bei einer Transplantat-Abstoßung.

**Revendications**

**1.** Protéine inactivant les ribosomes obtenue par extraction de graines de Vaccaria pyramidata selon le procédé suivant :

a) les graines sont homogénéisées avec du PBS (pH 7,15, NaCl 0,14 M/phosphate de sodium 5 mM), centrifugées, acidifiées avec de l'acide acétique glacial jusqu'à un pH de 4,0 - 4,1 et centrifugées;
b) le surnageant de l'étape (a) est purifié avec une colonne de S-Sepharose préparée avec de l'acétate de sodium 20 mM (pH 4,5), la colonne est lavée avec un volume dudit tampon précité puis avec du tampon phosphate de sodium 5 mM (pH 7,15);
c) la protéine est éluée avec ce même tampon contenant en plus du NaCl 1 M, dialysée contre du tampon phosphate de sodium 5 mM (pH 7,15) à 4°C et centrifugée;
d) la solution résultant de l'étape (c) est déposée sur une colonne de CM-Sepharose FF préparée avec le tampon de dialyse et lavée avec ce même tampon jusqu'à une absorbance inférieure à 0,1 avec 240 ml d'un gradient linéaire de 0 à 300 mM de NaCl;
e) les fractions présentant une activité inhibitrice sur les ribosomes sont réunies et dialysées contre de l'eau à 4°C;
f) la solution est concentrée sur membrane Amicon PM 10 (au moins 10 fois), diluée (au moins 10 fois) et concentrée;

cette protéine ayant une mobilité électrophorétique en SDS-PAGE sous forme de double bande d'environ 24000 Da, un poids moléculaire par filtration sur gel d'environ 29000 Da, un point isoélectrique $\geq$ 9,5 et la composition en acides aminés suivante :

| Lysine | 12,1 |
|---|---|
| Histidine | ---- |
| Arginine | 14,8 |
| Acide aspartique/asparagine | 22,0 |
| Thréonine | 12,0 |
| Sérine | 9,5 |
| Acide glutamique/glutamine | 19,0 |
| Proline | 6,9 |
| Glycine | 13,9 |
| Alanine | 13,4 |
| Cystéine | --- |

(suite)

| | |
|---|---|
| Valine | 12,3 |
| Méthionine | 2,1 |
| Isoleucine | 11,9 |
| Leucine | 15,1 |
| Tyrosine | 10,1 |
| Phénylalanine | 8,3 |
| Tryptophane | N.D. |

2. Protéine inactivant les ribosomes obtenue par extraction de graines de Saponaria ocymoides selon le procédé suivant :

a) les graines sont homogénéisées avec du PBS (pH 7,15, NaCl 0,14 M/phosphate de sodium 5 mM), centrifugées, acidifiées avec de l'acide acétique glacial jusqu'à un pH de 4,0 - 4,1 et centrifugées;
b) le sumageant de l'étape (a) est purifié avec une colonne de S-Sepharose préparée avec de l'acétate de sodium 20 mM (pH 4,5), la colonne est lavée avec un volume dudit tampon précité puis avec du tampon phosphate de sodium 5 mM (pH 7,15);
c) la protéine est éluée avec ce même tampon contenant en plus du NaCl 1 M, dialysée contre du tampon phosphate de sodium 5 mM (pH 7,15) à 4°C et centrifugée;
d) la solution résultant de l'étape (c) est déposée sur une colonne de CM-Sepharose FF préparée avec le tampon de dialyse et lavée avec ce même tampon jusqu'à une absorbance inférieure à 0,1 avec 1 l d'un gradient linéaire de 50 à 300 mM de NaCl;
e) les fractions présentant une activité inhibitrice sur les ribosomes sont réunies et dialysées contre de l'eau à 4°C;
f) purification par chromatographie d'interaction hydrophobe en dissolvant le produit résultant de l'étape (e) dans du sulfate d'ammonium à 30% (p/v), NaCl 0,5 M dans du phosphate de sodium 20 mM (pH 7,15) et traitement avec une colonne de Phenyl-Sepharose préparée avec ce même tampon, lavage jusqu'à une absorbance inférieure à 0,5 et élution de la protéine avec du PBS;

cette protéine ayant un poids moléculaire en SDS-PAGE de 30000 Da, un poids moléculaire par filtration sur gel de 30000 Da, un point isoélectrique ≥ 9,5 et la composition en acides aminés suivante:

| | |
|---|---|
| Lysine | 22,1 |
| Histidine | 2,7 |
| Arginine | 13,5 |
| Acide aspartique/asparagine | 33,2 |
| Thréonine | 12,7 |
| Sérine | 13,3 |
| Acide glutamique/glutamine | 19,0 |
| Proline | 7,8 |
| Glycine | 13,3 |
| Alanine | 20,3 |
| Cystéine | ---- |
| Valine | 14,5 |
| Méthionine | 4,8 |
| Isoleucine | 12,4 |
| Leucine | 21,1 |
| Tyrosine | 9,0 |
| Phénylalanine | 11,9 |
| Tryptophane | N.D. |

3. Procédé de purification de la protéine selon la revendication 1.

4. Procédé de purification de la protéine selon la revendication 2.

**5.** Conjugués des protéines selon les revendications 1 et 2 avec des hormones, des liposomes, des anticorps monoclonaux, des facteurs de croissance, des lectines, des cytokines, la transferrine, et peptides qui sont des fragments de ces protéines.

**6.** Conjugué selon la revendication 5 dans lequel l'anticorps est l'anticorps monoclonal Mint5, spécifique de EGF-R.

**7.** Conjugué selon la revendication 5 dans lequel l'anticorps est l'anticorps monoclonal MGR6, spécifique de HER2/neu.

**8.** Conjugués selon les revendications 6 et 7 obtenus par conjugaison chimique ou par recombinaison des gènes de la région variable de l'anticorps et de ceux codant pour la toxine.

**9.** Compositions pharmaceutiques contenant comme principe actif les protéines selon les revendications 1 et 2 en combinaison avec des supports pharmaceutiquement acceptables.

**10.** Compositions pharmaceutiques selon la revendication 9 à utiliser dans un traitement antiviral.

**11.** Utilisation des conjugués selon les revendications 5 à 8 pour la préparation de compositions pharmaceutiques utiles dans le traitement de tumeurs, de l'auto-immunité, d'infections, dans des traitements antiviraux et le rejet de greffons.

EP 0 717 753 B1

Fig. 1

Ocymoidine
CM-Sepharose elution profile

Absorbance at 280 nm

Inhibition of proteine synthesis (%)

Elution volume (ml)

Fig. 2

Piramidatine

CM-Sephardose elution profile

Elution volume (ml)

EP 0 717 753 B1

Fig. 3

Elution profile of purified Piramidatine

Reverse phase HPLC-chromatography

Chromatography in acetonitrile (0-70% in 50')
trifluoroacetate 0.1% buffer gradient

Fig. 4

Elution profile of purified Ocymoidine

Reverse phase HPLC-chromatography

Retention time (min)

Chromatography in acetonitrile (0-70% in 50')
trifluoroacetate 0.1% buffer gradient

Fig. 5

(A) (1) (2)(3) (4) (5) (6)

(A) = Reference molecular weight

(1) = Antibody MGR6

(2) = MGR6 - Ocymoidine

(3) = MGR6 - Piramidatine

(4) = Mint5 - Ocymoidine

(5) = Mint5 - Piramidatine

(6) = Antibody Mint5

Fig. 6

% inhib.

(A)

13.8ng/ml = $1.15 \times 10^{-9}$M

% inhib.

(B)

6.9 ng/ml = $4.57 \times 10^{-9}$M

Fig. 7

% inhib.

(A)

11.2 ng/ml = $0.93 \times 10^{-9}$M

% inhib.

(B)

11.45 ng/ml = $7.5 \times 10^{-9}$M